(19) Europäisches Patentamt
European Patent Office
Office européen des brevets

(11)    **EP 2 018 851 B1**

(12)    **EUROPEAN PATENT SPECIFICATION**

(45) Date of publication and mention
of the grant of the patent:
**17.07.2013   Bulletin 2013/29**

(51) Int Cl.:
*A61K 9/16* (2006.01)      *A61K 9/20* (2006.01)
*A61K 31/53* (2006.01)

(21) Application number: **07016160.9**

(22) Date of filing: **17.08.2007**

(54) **Enhanced formulations of lamotrigine**

Verbesserte Lamotrigin-Formulierungen

Formulations de lamotrigine améliorées

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **18.07.2007   US 779562**

(43) Date of publication of application:
**28.01.2009   Bulletin 2009/05**

(73) Proprietor: **Supernus Pharmaceuticals, Inc.
Rockville, MD 20850 (US)**

(72) Inventors:
• **Kidane, Argaw**
**Montgomery Village, Maryland 20886 (US)**
• **Edwards, Kevin**
**Virginia 20180 (US)**
• **Bhatt, Padmanabh P.**
**Rockville, Maryland 20850 (US)**

(74) Representative: **Louis Pöhlau Lohrentz
Patentanwälte
Postfach 30 55
90014 Nürnberg (DE)**

(56) References cited:
WO-A-96/17611          WO-A-03/104192
WO-A-2004/012741       US-A- 5 556 639
US-A1- 2005 123 606

• NYKANEN P ET AL: "Organic acids as excipients in matrix granules for colon-specific drug delivery" INTERNATIONAL JOURNAL OF PHARMACEUTICS (AMSTERDAM), vol. 184, no. 2, 20 July 1999 (1999-07-20), pages 251-261, XP002455708 ISSN: 0378-5173

Remarks:
The file contains technical information submitted after the application was filed and not included in this specification

**Description**

**BACKGROUND OF THE INVENTION**

WO 03/104192A2 is directed to a controlled release formulation of lamotrigine. WO 2004/012741 A1 is directed to a substained release formulation comprising lamotrigine.

Lamotrigine, having the chemical name 3,5-diamino-6-(2,3-dichlorophenyl)- as -triazine, is an anti-epileptic drug of the phenyltriazine class. The drug is typically used in both monotherapy and in adjunctive treatment with other antiepileptic agents for partial seizures and primary and secondary generalized tonic-clonic seizures in both adult and pediatric patients (i.e., children $\geq$ 2 years of age). Lamotrigine is also indicated for seizures associated with the Lennox-Gastaut syndrome and as maintenance treatment of Bipolar I Disorder to delay the time to occurrence of mood episodes (e.g. depression, mania, hypomania, mixed episodes) in patients treated for acute mood episodes with standard therapy.

Lamotrigine is currently available as an immediate-release ("IR") tablet as well as a chewable, dispersible tablet in various strengths from

GlaxoSmithKline under the brand name Lamictal™. In this form, lamotrigine is rapidly absorbed after oral administration with negligible first-pass metabolism (absolute bioavailability is 98%). Lamotrigine is metabolized predominantly by glucuronic acid conjugation, the major metabolite of lamotrigine being an inactive 2-N-glucuronide conjugate. IR formulations of lamotrigine, however, cause side effects associated with a rapid rise in the blood concentration of the drug and/or level of exposure.

"Modified"-release doses of lamotrigine (*i.e.*, doses other than IR, such as extended-release) could alleviate at least some of the side effects associated with immediate-release dosages. Because lamotrigine exhibits a decrease in solubility with increasing pH, the development of "modified"-release dosage forms of the drug has been problematic. When the release rate of a drug varies significantly with pH, the performance of a dosage form of the drug *in vivo* is influenced by the conditions of the gastro-intestinal tract and the residence time of the dosage form in the different segments of the gastrointestinal tract. In order to improve the release of drugs such as lamotrigine, the conventional wisdom has been to use acidifying agents, such as citric acid, in the formulation. However, this approach fails to enhance the release of lamotrigine in buffer media with pH greater than or equal to 6.8.

Hence, there is a need for a formulation of lamotrigine that exhibits a significantly similar release rate throughout the GI tract, irrespective of the pH of the environment. Advantageously, the formulation is an extended release formulation of lamotrigine; in particular, an extended release formulation of lamotrigine suitable for once-a-day administration.

**SUMMARY OF THE INVENTION**

The current invention addresses some of the aforementioned needs, in part, by providing a pharmaceutical formulation comprising lamotrigine or salts thereof admixed with a release equalizing composition according to claim 1. The composition comprises a combination of a release-enhancing polymer and a pharmaceutically acceptable organic acid.

In another embodiment of the invention, the lamotrigine formulation is an extended-release formulation. Extended release of lamotrigine may be achieved by incorporating a release-controlling polymer into the formulation. The resulting formulation exhibits a significantly similar release rate throughout the GI tract irrespective of the pH of the environment. The extended-release formulations of lamotrigine may be suitable for once-a-day administration, and take a variety of dosage forms, including tablets, pills, capsules, caplets, troches, sachets, cachets, pouches, and sprinkles.

Additionally, the invention is directed to the use of a pharmaceutical formulation for the manufacture of a pharmaceutical preparation for treatment of a neurological disorder in a mammalian subject according to claim 21 or 22.

**BRIEF DESCRIPTION OF THE DRAWINGS**

**Figure 1** shows the pH solubility profile for lamotrigine.

**Figure 2** shows the solubility of lamotrigine in the presence of select excipients.

**Figure 3** shows the dissolution profiles of control lamotrigine IR pellets (Formulation B) in acid (pH 1.1) and phosphate buffer (pH 6.8) dissolution media.

**Figure 4** shows the dissolution profiles of lamotrigine IR pellets (Formulation A) containing citric acid but not Eudragit L100-55.

**Figure 5** shows the dissolution profiles of Lamotrigine extended release tablets (Formulation C) according to one embodiment of the invention (containing enteric polymer and organic acid) in acid (pH 1.1), phosphate buffer (pH 6.8) dissolution media, and medium changeover where dissolution was carried out in an acid medium (pH 1.1) for the first 2 hours followed by changeover to phosphate buffer (pH 6.8) from 2-20 hours.

**Figure 6** shows the release profiles in phosphate buffer medium of three exemplary Lamotrigine extended release (CR-F, CR-M, and CR-S) prototypes.

**Figure 7** shows the human PK profiles of three exemplary prototypes (Lamotrigine CR-F, CR-M, and CR-S) whose dissolution profiles are shown in **Figure 6.**

**Figure 8** shows *in silico* steady state PK profiles for the three exemplary prototypes (Lamotrigine CR-F, CR-M, and CR-S).

## DETAILED DESCRIPTION OF THE INVENTION

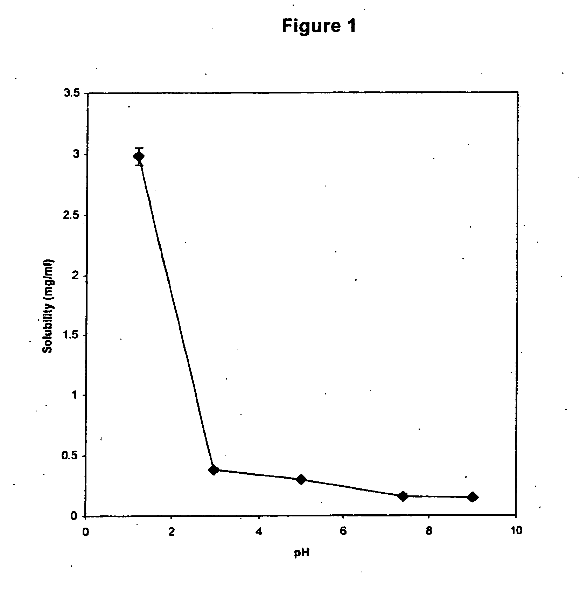

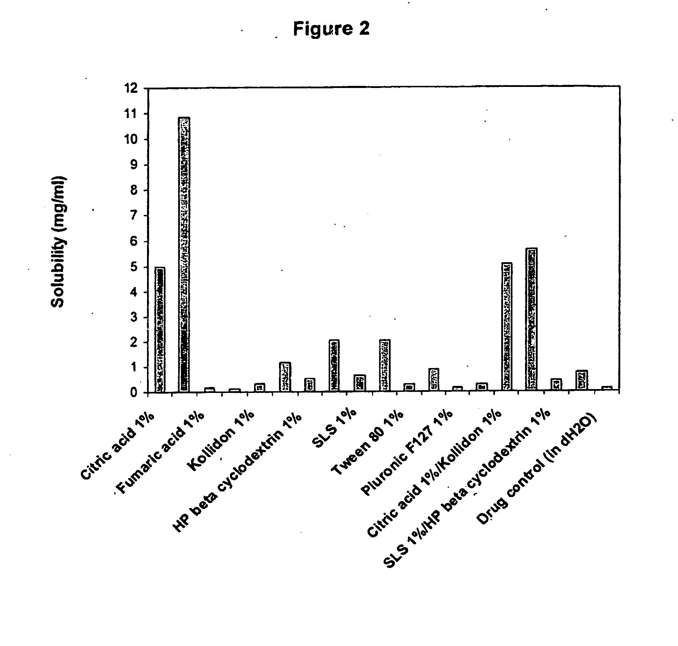

The solubility of lamotrgine is pH-dependent. **Figure 1** shows that lamotrigine is approximately 20 times more soluble at pH 1.2 than at pH 7.4 and **Figure 2** shows the effect of excipients on the drug's solubility. Taken together, these two figures suggest that the release rate of lamotrigine from a pharmaceutical formulation will be determined by or dependent upon pH. Hence, the "performance" (i.e., release rate) of a lamotrigine formulation *in vivo* will likely vary with the location of the formulation along segments of the GI tract—which have varying pH—and the residence time of the formulation in those segments. The variability of the release and performance is minimized by the formulations of the present invention.

As used herein, unless otherwise noted, "rate of release" or "release rate" of a drug refers to the quantity of drug released from a dosage form per unit time, e.g., milligrams of drug released per hour (mg/hr) or a percentage of a total drug dose released per hour. Drug release rates for dosage forms are typically measured as an in vitro rate of drug release, i.e., a quantity of drug released from the dosage form per unit time measured under appropriate conditions and in a suitable fluid.

The present inventors unexpectedly discovered that mixing lamotrigine with a "release-equalizing" composition, comprising a combination of a release-enhancing polymer and an organic acid, results in a lamotrigine formulation that exhibits (compared to an IR formulation of equivalent dose) enhanced solubility, enhanced dissolution and a significantly similar rate of release throughout the gastro-intestinal ("GI") tract, irrespective of the pH of the environment. A "release-equalizing" composition as defined herein is a composition that provides a significantly similar rate of release throughout the "GI" tract. For the purposes of this invention, the term "significantly similar release rate means that the similarity factor $f_2$ between the profile at the low pH and the profile at high pH is at least 50 (see "Guidance for Industry: Dissolution testing of Immediate Release Solid Oral Dosage Forms", U.S. Department of Health and Human Services, Food and Drug Administration Center for Drug Evaluation and Research (CDER), August 1997, for the definition of $f_2$). The similarity factor ($f_2$) is a logarithmic reciprocal square root transformation of the sum of squared error and is a measurement of the similarity in the percent (%) dissolution between the two curves:

$$f_2 = 50 \log\{[1+(1/n)\textstyle\sum_1^n (R - T)^2]^{-0.5} * 100\}$$

Release-equalizing compositions of the present invention comprise a pharmaceutically acceptable organic acid and a release-enhancing polymer. Any pharmaceutically acceptable organic acid, as well as combinations of such acids, may be used in the practice of the current invention. Without any limitations thereon, suitable acids may be citric acid, fumaric acid, tartaric acid, adipic acid, succinic acid, and maleic acid, of which fumaric and citric acids are preferred. The acid may be incorporated into the release-equalizing formulation in the amount of from 1 to 20 wt %, preferably in the amount of from 5 to 20 wt %.

The release-enhancing polymers are enteric polymers soluble at pH $\geq$ 4.5. Such polymers include, but are not limited to, cellulose acetate phthalate, cellulose acetate succinate, methylcellulose phthalate, ethylhydroxycellulose phthalate, polyvinylacetate phthalate, polyvinylbutyrate acetate, vinyl acetate-maleic anhydride copolymer, styrene-maleic monoester copolymer, methyl acrylate-methacrylic acid copolymer, and methacrylate-methacrylic acid-octyl acrylate copolymer. In a preferred embodiment of the invention, Eudragit L100-55 is used as a release-enhancing polymer. The release-enhancing polymers may be incorporated into the release-equalizing formulation in the amount of from 5 to 50 wt %; preferably in the amount of from 10 to 35 wt %.

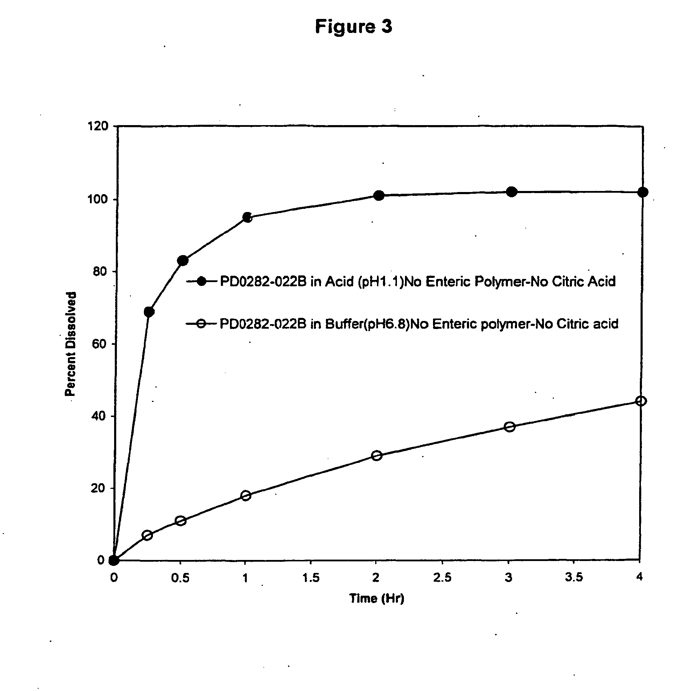

**Figure 3** shows dissolution profiles for formulations of lamotrigine without a release-equalizing composition in acid (pH 1.1) or phosphate buffer (pH 6.8) dissolution media. As expected, the profiles vary significantly with pH.

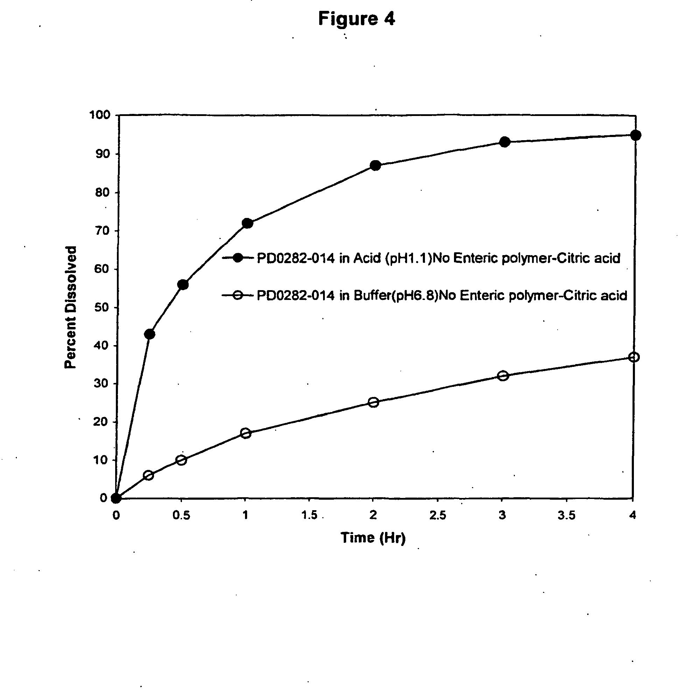

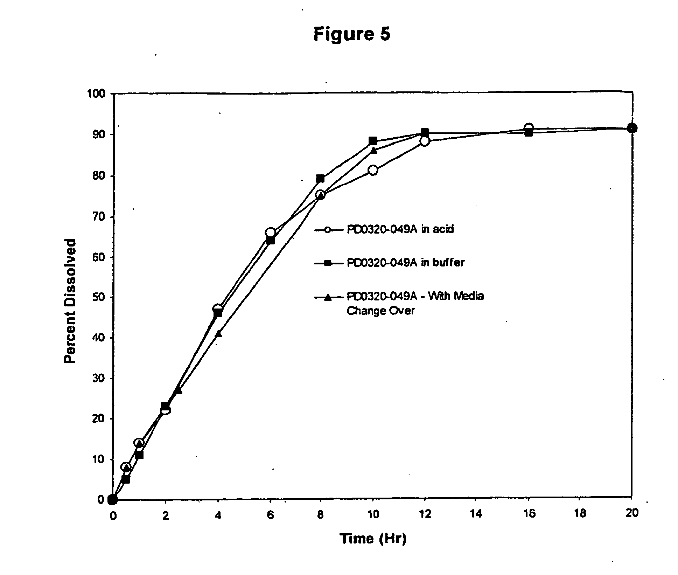

**Figure 4** shows dissolution profiles for lamotrigine in a release-equalizing composition that lacks a release-enhancing enteric polymer. There is a significant difference between the dissolution profile in acid (pH 1.1) versus phosphate buffer (pH 6.8) media. **Figure 5,** by contrast, shows dissolution profiles for lamotrigine in release-equalizing formulations as taught by the present invention (i.e., containing both an organic acid and an enteric polymer). Note that the release of lamotrigine from this formulation is similar in either dissolution media, highlighting the pH-independence of the inventive formulations. Also shown in **Figure 5** is the dissolution profile for the same formulation with media changeover, where the dissolution is carried out in acid (pH 1.1) medium for the first 2 hours followed by media changeover to phosphate buffer (pH 6.8). The profile is similar to those obtained in acid and phosphate buffer media.

In a further embodiment of the invention, a "release-controlling" polymer may be additionally included into formulations of the current invention to provide "extended-release" formulations of lamotrigine that have a significantly similar release rate throughout the GI tract. An extended-release formulation (also referred to herein as "controlled release" or "CR") is one that releases 80% of its drug *in vitro* ($T_{80}$) over a span of at least 2 hours.

Extended-release formulations of lamotrigine may have the release-controlling polymer(s) admixed with other ingredients into a release-equalizing formulation (e.g., organic acid and release-enhancing polymer), or may be supplied via a coating on a lamotrigine-containing matrix. Release-controlling polymers such as ethylcellulose, Eudragit RL, Eudragit RS, cellulose acetate, hydroxypropylmethylcellulose (HPMC), hydroxypropylcellulose (HPC), hydroxyethylcellulose (HEC), methylcellulose (MC), and PVA-PEG copolymer can be used to form an extended-release coating.

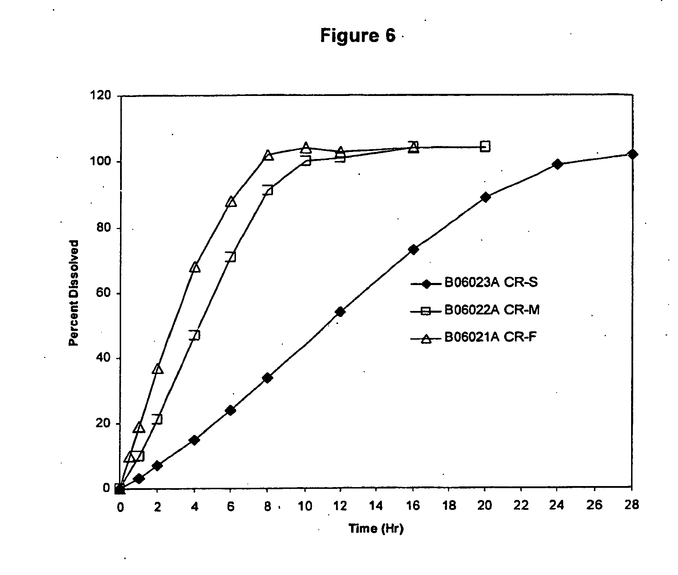

The present inventors also surprisingly discovered that when a release-controlling polymer is incorporated into a release-equalizing composition, the release-controlling polymer not only controls the rate of release, but also improves the effectiveness of the release-equalizing composition in a synergistic manner. The degree of synergism is determined by the nature and molecular weight of the release controlling polymer, as can be seen in Example 2 and **Figure 6**, discussed below.

Polymers suitable for such synergistic action may be selected from a group consisting of hydroxypropylmethylcellulose (HPMC), hydroxypropylcellulose (HPC), hydroxyethylcellulose (HEC), methylcellulose (MC), powdered cellulose such as microcrystalline cellulose, cellulose acetate, sodium carboxymethylcellulose, calcium salt of carboxymethylcellulose, and ethylcellulose; alginates, gums such as guar and xanthan gums; cross-linked polyacrylic acid derivatives such as Carbomers (aka Carbopol™), that are available in various molecular weight grades from Noveon Inc. (Cincinnati, Ohio); carageenan; polyvinyl pyrrolidone and its derivatives such as crospovidone; polyethylene oxides; and polyvinyl alcohol.

The release-controlling polymer may be incorporated into the formulation in the amount of up to 50 % by weight. In a particular embodiment of the invention, the release-controlling polymer is polyethylene oxide, preferably of the Polyox™ class. Polyox™ are water soluble linear polyethylene oxide polymers available from Dow Chemical.

As may be seen from Examples 2 and 3 and **Figure 6**, the release profile of the inventive lamotrigine compositions can be selectively adjusted by utilizing polyethylene oxide of different molecular weights. This level of adjustability provided by the current invention allows the development of extended-release formulations of lamotrigine that provide a better fit to the goals and modalities of treatment.

Extended-release formulations have an advantage over IR formulations in that the level of side-effects such as dizziness, ataxia, somnolence, headache, diplopia, blurred vision, nausea, vomiting, and rash, associated with rapid rise in the blood concentration of the drug when given in an IR form, is diminished. Fewer side-effects are known to lead to better treatment and compliance on the part of the patient.

In yet another embodiment, extended-release formulations of lamotrigine according to the invention provide a pharmacokinetic profile such that a maximum steady-state plasma concentration (Cmax) is in the range of from $C_{minIR}$ to 110% of $C_{maxIR}$, wherein $C_{minIR}$ and $C_{maxIR}$ are the minimum and the maximum plasma concentrations respectively produced by the same amount of lamotrigine administered BID. In another embodiment, extended-release formulations of lamotrigine according to the invention provide a pharmacokinetic profile such that a relative steady state AUC is in the range of from 80% to 125% of an $AUC_{IR}$, wherein $AUC_{IR}$ is an area under the curve produced by the same amount of lamotrigine administered as an immediate-release formulation BID.

For the purposes of this invention, BID administration is defined as administration of the same amount of lamotrigine in two equal doses twelve hours apart. Further, AUC is defined here as the area under the plasma concentration-time curve. the AUC is directly proportional to the total amount of unmetabolized drug that reaches the systemic circulation.

Extended-release formulations of lamotrigine may be designed for oncea-day administration, though other modes of administration, such as twice-a-day, are also possible. The formulations of the current invention may be incorporated in any solid oral dosage form including, but not limited to, a tablet, a capsule, a caplet, a troche, a sachet, a cachet, a pouch, and sprinkles. The amount of lamotrigine in the dosage form *may* vary from 5 mg to 500 mg.

The formulations of the current invention may be used for the treatment or prevention of all disorders or conditions of a mammalian subject for which lamotrigine treatment is indicated or desirable. Without putting any limitations thereon, such disorders include epilepsy, Lennox-Gastaut syndrome, and bipolar disorder.

The formulations of the current invention may be prepared by a variety of methods well known in the art and include a variety of standard pharmaceutical excipients such as fillers, binders, lubricants, and others.

## EXAMPLES

### Example I. Control lamotrigine immediate-release ("IR") pellets

**Table 1** provides the composition of two control formulations (Formulation A and Formulation B) of lamotrigine IR pellets. Formulations A and B were prepared by mixing dry components in KG-5 high shear granulator (Key International, Englishtown, NJ). Granules were then extruded using DG-L2 Dome granulator (LCI Corporation, Charlotte, NC). Extrudates were spheronized using QJ-400G spheronizer (LCI Corporation, Charlotte, NC). Pellets were dried in an oven at 40°C overnight. Dissolution tests were carried out in acid (pH 1.1) and phosphate buffer (pH 6.8) media. Dissolution profiles are shown in **Figures 3** and **4.** Both formulations exhibited pH dependent release profiles.

| Table 1. Composition of two lamotrigine IR pellets (%w/w). | | |
|---|---|---|
| Components | PD0282-014 Formulation A | PD0282-022B Formulation B |
| Lamotrigine | 50 | 50 |
| SMCC50 | 30 | 45 |
| Lactose | 5 | |
| Kollidon 25 | 5 | 5 |
| Sodium Lauryl Sulfate | 5 | - |
| Maltodextrin (Maltrin M150) | 5 | - |
| Citric Acid | 5 | - |
| Total | 100 | 100 |

### Example II. Lamotrigine tablet composition of the invention

**Table 2** provides the composition of Formulation C according to the invention. The formulation was granulated using a Key high shear granulator with water as a granulating liquid. The drug and excipients were added to the bowl, and dry blended for about 1 minute at a blade speed of ~260RPM. A total of 112g of deionized water was added to achieve granulation. The lot was dried in a fluid bed dryer (GPCG-1) to a final product temperature of no more than 50°C. The moisture content of the granulation was determined using a moisture analyzer, and was found to be 1.53% by weight. The dried granules were screened through an 18 mesh sieve, blended with magnesium stearate and tableted on a rotary tablet press. The target tablet weight and hardness were 333 mg and 8-10 kp, respectively. Tablet dissolution was tested in acid (pH 1.1), phosphate buffer (pH 6.8), and media change-over where the dissolution medium was changed from acid to buffer at the two hour time point during the dissolution run. The dissolution profiles are shown in **Figure 5**.

| Table 2. Composition for Lot PD0320-049A (Matrix Tablets). Formulation C | |
|---|---|
| Component | (%w/w) |
| Lamotrigine | 30 |
| Prosolv SMCC50 | 10 |
| Eudragit L100-55 | 25 |
| Fumaric Acid | 5 |
| Polyox WSR N80 | 25 |
| Kollidon 25 | 5 |
| Magnesium Stearate | 0.5 |
| Batch Size (g) | 500 |

**EXAMPLE III. Lamotrigine extended release (CR-F, CR-M, and CR-S) clinical batches.**

Three prototypes of the invention were manufactured to provide fast, medium and slow extended release profiles (Lamotrigine CR-F, CR-M, and CRS, respectively). The composition for these prototypes is given in **Table 3.** These prototypes were manufactured following the same processing steps as provided in Example II. The products were used in a human pharmacokinetic (PK) study.

| Table 3. Composition of Lamotrigine CR-F, CR-M and CR-S Clinical Batches | | | |
|---|---|---|---|
| Formulation | B06021 (CR-F) | B06022 (CR-M) | B06023 (CR-S) |
| | %w/w per dosage unit | | |
| Lamotrigine | 30.0 | 30.0 | 30.0 |
| Prosolv SMCC50 | 9.5 | 9.5 | 9.5 |
| Polyethylene Oxide, NF (Polyox WSR N80) | 25.0 | - | - |
| Polyethylene Oxide, NF (Polyox WSR 1105) | - | 25.0 | - |
| Polyethylene Oxide, NF (Polyox WSR 301) | - | - | 25.0 |
| Methacrylic Acid Copolymer, Type C, NF (Eudragit L100-55) | 25.0 | 25.0 | 25.0 |
| Fumaric Acid, Fine Granular | 5.0 | 5.0 | 5.0 |
| Povidone, USP (Kollidon 25) | 5.0 | 5.0 | 5.0 |
| Magnesium Stearate, NF (PH Vegetable) | 0.5 | 0.5 | 0.5 |
| Batch Size | 100.0 | 100.0 | 100.0 |

**Figure 6** shows the dissolution profiles for these prototypes in phosphate buffer medium (pH 6.8).

**Example IV. Human pilot pharmacokinetic study and *in silico* modeling**

Human pilot pharmacokinetic studies were conducted to assess the pharmacokinetics of the once-a-day, 100-mg dose of the three exemplary lamotrigine extended-release (CR-F, CR-M, CR-S) tablets, and a twice-a-day 50-mg dose of immediate release Lamictal® tablets (2x25mg tablets), for a total daily dose of 100 mg, of under fasting conditions.

| Table 4. Drug formulations | |
|---|---|
| Formulation | Dose |
| Lamotrigine CR-F Tablets, 100 mg | $1 \times 100$ mg |
| Lamotrigine CR-M Tablets, 100 mg | $1 \times 100$ mg |
| Lamotrigine CR-S Tablets, 100 mg | $1 \times 100$ mg |
| Lamictal® Tablets, 25mg | $2 \times 25$ mg (BID) |

Blood samples ($1 \times 3$ mL) were collected at time 0, the time of dose administration (within 30-minute window prior to dose) and at (for Treatments A, B, and C) Time 0.25, 0.5, 1,1.5, 2, 3, 4, 5, 6, 8, 10, 12, 16, 18, 24, 36, 48, 60, 72 and 96 hours post-dose for the three exemplary controlled release products. Blood collection times for the reference product were at Time 0, the time of dose administration (within 30-minute window prior to dose), and at Time 0.25, 0.5, 0.75, 1, 1.5, 2, 2.5, 3, 4, 5, 6, 8, 11.833 (up to 10 minutes prior to the second dose administration), 12.5, 13, 13.5, 14, 14.5, 15, 16, 17, 18, 20, 24, 36, 48, 60, 72 and 96 hours post-first-dose. Plasma samples were analyzed using an LC-MS instrument.

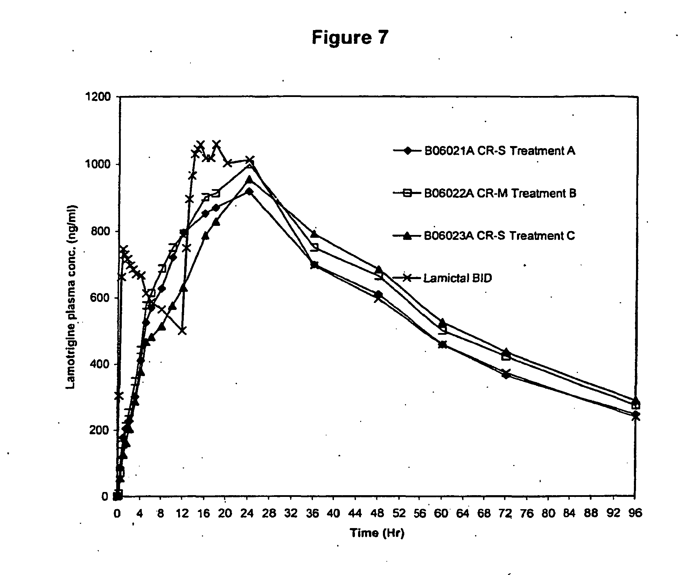

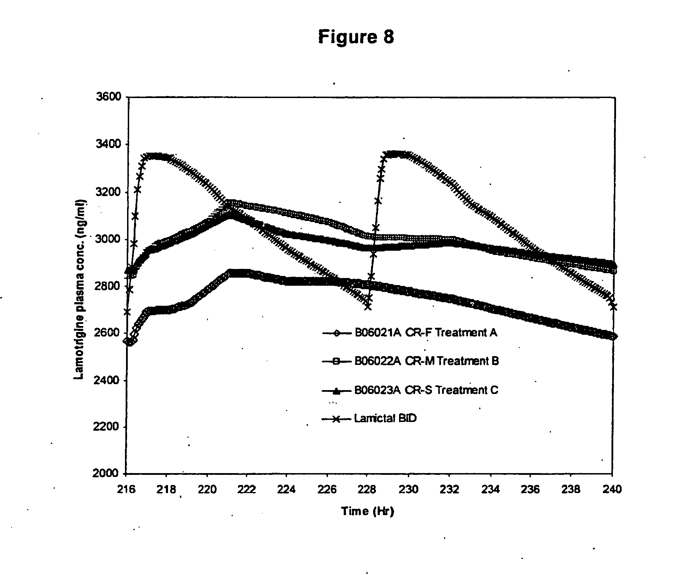

**Figure 7** shows the plasma lamotrigine concentration versus time profiles for the three extended release products and the reference product. Shown in **Figure 8** is the *in silico* steady state plasma lamotrigine concentration versus time profiles.

**Claims**

1. A pharmaceutical formulation of lamotrigine comprising lamotrigine or salts thereof admixed with a release-equalizing composition comprising a pharmaceutically acceptable organic acid and a release-enhancing polymer, said formulation exhibiting a significantly similar rate of release with a similarity factor of at least 50 throughout the GI tract, , wherein the release enhancing polymer is an enteric polymer, which is soluble at pH $\geq$ 4.5.

2. The formulation of claim 1, wherein said enteric polymer is selected from the group consisting of, cellulose acetate succinate, methylcellulose phthalate, ethylhydroxycellulose phthalate, polyvinylacetate phthalate, polyvinylbutyrate acetate, vinyl acetate-maleic anhydride copolymer, styrene-maleic monoester copolymer, methyl acrylate-methacrylic acid copolymer, and methacrylate-methacrylic acid-octyl acrylate copolymer.

3. The formulation of claim 1, wherein said enteric polymer is Eudragit L100-55.

4. The formulation of claim 1, wherein said organic acid is selected from the group consisting of citric acid, fumaric acid, tartaric acid, adipic acid, succinic acid, and maleic acid.

5. The formulation of claim 4, wherein said organic acid is citric acid or fumaric acid.

6. The formulation of claim 1 which is an extended-release formulation.

7. The formulation of claim 6, wherein said formulation provides for a maximum steady state plasma concentration (Cmax) in the range from $C_{minIR}$ to 110% of $C_{maxIR}$, wherein $C_{minIR}$ and $C_{maxIR}$ are the minimum and the maximum plasma concentrations respectively produced by the same amount of lamotrigine administered as an immediate-release formulation BID.

8. The formulation of claim 6, wherein said formulation provides for a relative steady state AUC in the range of from 80% to 125% of an $AUC_{IR}$, wherein $AUC_{IR}$ is an area under the curve produced by the same amount of lamotrigine administered as an immediate release formulation BID.

9. The formulation of claim 6 further comprising a coating of a release-controlling polymer selected from a group consisting of ethylcellulose, Eudragit RL, Eudragit RS, cellulose acetate, hydroxypropylmethylcellulose HPMC, hydroxyethylcellulose (HEC), methylcellulose (MC), and PVA-PEG copolymer.

10. The formulation of claim 6 wherein said release-equalizing composition further comprises a release-controlling polymer selected from a group consisting of hydroxypropylmethylcellulose (HPMC), hydroxypropylcellulose (HPC), hydroxyethylcellulose (HEC), methylcellulose (MC), powdered cellulose, cellulose acetate, sodium carboxymethylcellulose, calcium salt of carboxymethylcellulose and ethylcellulose; alginates, guar gum, xanthan gum; cross-linked polyacrylic acid derivatives; carageenan; polyvinyl pyrrolidone and its derivatives; polyethylene oxides; and polyvinyl alcohol.

11. The formulation of claim 10, wherein said release controlling-polymer is polyethylene oxide.

12. The formulation of claim 1 comprising from 5 to 500 mg of lamotrigine or salts thereof.

13. The formulation of claim 1, comprising from 5 to 20 wt % organic acid.

14. The formulation of claim 1, comprising from 5-50 wt % release-enhancing polymer.

15. The formulation of claim 10, comprising up to 50 wt % release-controlling polymer.

16. The formulation of claim 6 suitable for once-a-day administration.

17. The formulation of anyone of claims 1 to 16, wherein the release-equalizing composition comprises the organic acid and the realease-enhancing polymer and is admixed with lamotrigine to form a matrix.

18. The formulation of claim 17, wherein the release controlling polymer is admixed into the matrix and has a dual function of controlling a rate of release, and equalizing the release profile synergistically with the organic acid and the release enhancing polymer.

19. The formulation of claim 17, wherein said release controlling polymer is coated onto the matrix.

20. The pharmaceutical formulation according to anyone of claims 1 to 19 provided as a dosage form selected from a group consisting of a tablet, a pill, a capsule, a caplet, a troche, a sachet, a cachet, a pouch, and sprinkles.

21. Use of a pharmaceutical formulation according to any one of claims 1 to 20 for the manufacture of a pharmaceutical preparation for treating a neurological disorder.

22. The use of claim 21, wherein said neurological disorder is selected from a group consisting of epilepsy, Lennox-Gastaut syndrome, and a bipolar disorder.

23. The formulation according to anyone of claims 1 to 20, which is an extended-release formulation of lamotrigine that exhibits a significantly similar release rate with a similarity factor of at least 50 throughout the GI tract irrespective of the pH of the environment, said formulation comprising a therapeutically effective amount of lamotrigine or salts thereof, fumaric acid, Eudragit L100-55, and polyethylene oxide, admixed together and forming a matrix.

**Patentansprüche**

1. Pharmazeutische Zubereitung von Lamotrigin, umfassend Lamotrigin oder Salze davon im Gemisch mit einer die Freisetzung egalisierenden Zusammensetzung, umfassend eine pharmazeutisch verträgliche organische Säure und ein die Freisetzung verstärkendes Polymeres, wobei die Zubereitung eine signifikant ähnliche Freisetzungsgeschwindigkeit mit einem Ähnlichkeitsfaktor von mindestens 50 im gesamten GI-Trakt aufweist, wobei das die Freisetzung verstärkende Polymere ein enterisches Polymeres ist, das bei einem pH-Wert $\geq 4,5$ löslich ist.

2. Zubereitung nach Anspruch 1, wobei das enterische Polymere ausgewählt ist aus der Gruppe, die besteht aus Celluloseacetosuccinat, Methylcellulosephthalat, Ethylhydroxycellulosephthalat, Polyvinylacetatphthalat, Polyvinylbutyratacetat, einem Vinylacetat-Maleinsäureanhydrid-Copolymeren, einem Styrol-Maleinsäuremonoester-Copolymeren, einem Methylacrylat-Methacrylsäure-Copolymeren und einem Methacrylat-Methacrylsäure-Octylacrylat-Copolymeren.

3. Zubereitung nach Anspruch 1, wobei es sich beim enterischen Polymeren um Eudragit L100-55 handelt.

4. Zubereitung nach Anspruch 1, wobei die organische Säure aus der Gruppe ausgewählt ist, die besteht aus Citronensäure, Fumarsäure, Weinsäure, Adipinsäure, Bernsteinsäure und Maleinsäure.

5. Zubereitung nach Anspruch 4, wobei es sich bei der organischen Säure um Citronensäure oder Fumarsäure handelt.

6. Zubereitung nach Anspruch 1, wobei es sich um eine Zubereitung mit verlängerter Freisetzung handelt.

7. Zubereitung nach Anspruch 6, wobei die Zubereitung für eine maximale stationäre Plasmakonzentration (Cmax) im Bereich von $C_{minIR}$ bis 110 % von $C_{maxIR}$ sorgt, wobei $C_{minIR}$ und $C_{maxIR}$ die minimale bzw. maximale Plasmakonzentration bedeuten, die durch die gleiche Menge Lamotrigin, das als eine BID-Zubereitung mit sofortiger Freisetzung verabreicht wird, erzeugt werden.

8. Zubereitung nach Anspruch 6, wobei die Zubereitung für einen relativen stationären AUC-Wert von 80 bis 125 % eines $AUC_{IR}$-Werts sorgt, wobei $AUC_{IR}$ eine Fläche unter der Kurve bedeutet, die durch die gleiche Menge an Lamotrigin, das als eine BID-Zubereitung mit sofortiger Freisetzung verabreicht wird, erzeugt wird.

9. Zubereitung nach Anspruch 6, ferner umfassend einen Überzug aus einem die Freisetzung kontrollierenden Polymeren, das aus einer Gruppe ausgewählt ist, die besteht aus Ethylcellulose, Eudragit RL, Eudragit RS, Celluloseacetat, Hydroxypropylmethylcellulose (HPMC), Hydroxyethylcellulose (HEC), Methylcellulose (MC) und einem PVA-PEG-Copolymeren.

10. Zubereitung nach Anspruch 6, wobei die die Freisetzung egalisierende Zusammensetzung ferner ein die Freisetzung kontrollierendes Polymeres umfasst, das aus einer Gruppe ausgewählt ist, die besteht aus Hydroxypropylmethylcellulose (HPMC), Hydroxypropylcellulose (HPC), Hydroxyethylcellulose (HEC), Methylcellulose (MC), pulverförmiger Cellulose, Celluloseacetat, Natriumcarboxymethylcellulose, Calciumsalz von Carboxymethylcellulose und Ethylcellulose; Alginaten, Guargummi, Xanthangummi; vernetzten Polyacrylsäurederivaten; Carageenan, Polyvinylpyrrolidon und Derivaten davon; Polyethylenoxiden; und Polyvinylalkohol.

11. Zubereitung nach Anspruch 10, wobei es sich bei dem die Freisetzung kontrollierenden Polymeren um Polyethylenoxid handelt.

12. Zubereitung nach Anspruch 1, umfassend 5 bis 500 mg Lamotrigin oder Salze davon.

13. Zubereitung nach Anspruch 1, umfassend 5 bis 20 Gew.-% organische Säure.

14. Zubereitung nach Anspruch 1, umfassend 5 bis 50 Gew.-% des die Freisetzung verstärkenden Polymeren.

15. Zubereitung nach Anspruch 10, umfassend bis zu 50 Gew.-% des die Freisetzung verstärkenden Polymeren.

16. Zubereitung nach Anspruch 6, geeignet für eine einmalige tägliche Verabreichung.

17. Zubereitung nach einem der Ansprüche 1 bis 16, wobei die die Freisetzung egalisierende Zusammensetzung die organische Säure und das die Freisetzung verstärkende Polymere umfasst und mit Lamotrigin unter Bildung einer Matrix vermischt wird.

18. Zubereitung nach Anspruch 17, wobei das die Freisetzung kontrollierende Polymere in die Matrix eingemischt wird und eine doppelte Funktion zur Kontrolle der Freisetzungsgeschwindigkeit und zur Egalisierung des Freisetzungsprofils in synergistischer Weise mit der organischen Säure und dem die Freisetzung verstärkenden Polymeren aufweist.

19. Zubereitung nach Anspruch 17, wobei das die Freisetzung kontrollierende Polymere als Überzug auf die Matrix aufgetragen ist.

20. Pharmazeutische Zubereitung nach einem der Ansprüche 1 bis 19, bereitgestellt als Dosierungsform, die aus der Gruppe ausgewählt ist, die aus einer Tablette, einer Pille, einer Kapsel, einem Kaplet, einer Pastille, einem Sachet, einem Cachet, einem Beutel und einem Sprühmittel besteht.

21. Verwendung einer pharmazeutischen Zubereitung nach einem der Ansprüche 1 bis 20 zur Herstellung eines pharmazeutischen Präparats zur Behandlung einer neurologischen Störung.

22. Verwendung nach Anspruch 21, wobei die neurologische Störung aus einer Gruppe ausgewählt ist, die besteht aus Epilepsie, Lennox-Gastaut-Syndrom und einer bipolaren Störung.

23. Zubereitung nach einem der Ansprüche 1 bis 20, bei der es sich um eine Zubereitung mit verlängerter Freisetzung von Lamotrigin handelt, die eine signifikant ähnliche Freisetzungsgeschwindigkeit mit einem Ähnlichkeitsfaktor von mindestens 50 im gesamten GI-Trakt unabhängig vom pH-Wert der Umgebung aufweist, wobei die Zubereitung eine therapeutisch wirksame Menge an Lamotrigin oder Salzen davon, Fumarsäure, Eudragit L100-55 und Polyethylenoxid im Gemisch miteinander und unter Bildung einer Matrix umfasst.

**Revendications**

1. Formulation pharmaceutique de lamotrigine comprenant de la lamotrigine ou ses sels mélangés avec une composition d'égalisation de libération comprenant un acide organique pharmaceutiquement acceptable et un polymère accentuant la libération, ladite formulation présentant une vitesse de libération significativement similaire avec un facteur de similarité d'au moins 50 sur la totalité du tractus gastro-intestinal, dans laquelle le polymère accentuant la libération est un polymère entérique, qui est soluble à pH $\geq$ 4,5.

2. Formulation selon la revendication 1, dans laquelle ledit polymère entérique est choisi dans le groupe constitué par l'acétate succinate de cellulose, le phtalate de méthylcellulose, le phtalate d'éthylhydroxycellulose, le poly(acétate phtalate de vinyle), le poly(butyrate acétate de vinyle), un copolymère d'acétate de vinyle-anhydride maléique, un copolymère de styrène-monoester maléique, un copolymère d'acrylate de méthyle-acide méthacrylique et un copolymère de méthacrylate-acide méthacrylique-acrylate d'octyle.

3. Formulation selon la revendication 1, dans laquelle ledit polymère entérique est l'Eudragit L100-55.

4. Formulation selon la revendication 1, dans laquelle ledit acide organique est choisi dans le groupe constitué par l'acide citrique, l'acide fumarique, l'acide tartrique, l'acide adipique, l'acide succinique et l'acide maléique.

5. Formulation selon la revendication 4, dans laquelle ledit acide organique est l'acide citrique ou l'acide fumarique.

6. Formulation selon la revendication 1, qui est une formulation à libération prolongée.

7. Formulation selon la revendication 6, dans laquelle ladite formulation permet une concentration dans le plasma maximale en régime permanent (Cmax) dans la gamme de $C_{minIR}$ à 110 % de $C_{maxIR}$, où $C_{minIR}$ et $C_{maxIR}$ sont respectivement les concentrations dans le plasma minimale et maximale produites par la même quantité de lamotrigine administrée sous forme de formulation à libération immédiate deux fois par jour.

8. Formulation selon la revendication 6, dans laquelle ladite formulation permet une surface sous la courbe (SSC) relative en régime permanent dans la gamme de 80 % à 125 % d'une $SSC_{IR}$, où $SSC_{IR}$ est une surface sous la courbe produite par la même quantité de lamotrigine administrée sous forme de formulation à libération immédiate deux fois par jour.

9. Formulation selon la revendication 6, comprenant en outre un revêtement d'un polymère de régulation de libération choisi dans le groupe constitué par l'éthylcellulose, l'Eudragit RL, l'Eudragit RS, l'acétate de cellulose, l'hydroxypropylméthylcellulose HPMC, l'hydroxyéthylcellulose (HEC), la méthylcellulose (MC) et un copolymère de PVA-PEG.

10. Formulation selon la revendication 6, dans laquelle ladite composition d'égalisation de libération comprend en outre un polymère de régulation de libération choisi dans le groupe constitué par l'hydroxypropylméthylcellulose (HPMC), l'hydroxypropylcellulose (HPC), l'hydroxyéthylcellulose (HEC), la méthylcellulose (MC), la cellulose en poudre, l'acétate de cellulose, la carboxyméthylcellulose de sodium, le sel de calcium de carboxyméthylcellulose et d'éthylcellulose ; les alginates, la gomme de guar, la gomme de xanthane ; les dérivés réticulés de poly(acide acrylique) ; la carraghénine ; la poly(vinyl pyrrolidone) et ses dérivés ; les poly(oxydes d'éthylène) ; et le poly(alcool vinylique).

11. Formulation selon la revendication 10, dans laquelle ledit polymère de régulation de libération est le poly(oxyde d'éthylène).

12. Formulation selon la revendication 1, comprenant de 5 à 500 mg de lamotrigine ou de ses sels.

13. Formulation selon la revendication 1, comprenant de 5 à 20 % en poids d'un acide organique.

14. Formulation selon la revendication 1, comprenant de 5 à 50 % en poids de polymère accentuant la libération.

15. Formulation selon la revendication 10, comprenant jusqu'à 50 % en poids de polymère de régulation de libération.

16. Formulation selon la revendication 6, appropriée pour une administration une fois par jour.

17. Formulation selon l'une quelconque des revendications 1 à 16, dans laquelle la composition d'égalisation de libération comprend l'acide organique et le polymère accentuant la libération et est mélangée avec de la lamotrigine pour former une matrice.

18. Formulation selon la revendication 17, dans laquelle le polymère de régulation de libération est mélangé dans la matrice et a la fonction double de réguler une vitesse de libération et d'égaliser le profil de libération de manière synergique avec l'acide organique et le polymère accentuant la libération.

19. Formulation selon la revendication 17, dans laquelle ledit polymère de régulation de libération est revêtu sur la matrice.

20. Formulation pharmaceutique selon l'une des revendications 1 à 19, à condition qu'une forme galénique soit choisie dans le groupe constitué par un comprimé, une pilule, une capsule, un comprimé-capsule, une tablette, un sachet, un cachet, une poche et des gouttes.

21. Utilisation d'une formulation pharmaceutique selon l'une quelconque des revendications 1 à 20, pour la fabrication d'une préparation pharmaceutique destinée à traiter un trouble neurologique.

22. Utilisation selon la revendication 21, dans laquelle ledit trouble neurologique est choisi dans le groupe constitué par l'épilepsie, le syndrome de Lennox-Gastaut et un trouble bipolaire.

23. Formulation selon l'une quelconque des revendications 1 à 20, qui est une formulation à libération prolongée de lamotrigine qui présente une vitesse de libération significativement similaire avec un facteur de similarité d'au moins 50 sur la totalité du tractus gastro-intestinal indépendamment du pH de l'environnement, ladite formulation comprenant une quantité thérapeutiquement efficace de lamotrigine ou de ses sels, de l'acide fumarique, de l'Eudragit L100-55 et du poly(oxyde d'éthylène), mélangés ensemble et formant une matrice.

**Figure 1**

## Figure 2

# Figure 3

# Figure 4

## Figure 5

## Figure 6

## Figure 7

## Figure 8

**EP 2 018 851 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03104192 A2
- WO 2004012741 A1